(19) Europäisches Patentamt
European Patent Office
Office européen des brevets

(11) Publication number: 0 116 938 A1

(12) EUROPEAN PATENT APPLICATION

(21) Application number: 84101493.9

(22) Date of filing: 14.02.84

(51) Int. Cl.³: C 07 D 307/86
C 07 D 263/56, C 07 D 209/08
C 07 D 333/54, A 61 K 31/325

(30) Priority: 14.02.83 US 466285

(43) Date of publication of application: 29.08.84 Bulletin 84/35

(84) Designated Contracting States: AT BE CH DE FR GB IT LI LU NL SE

(71) Applicant: USV PHARMACEUTICAL CORPORATION
1 Scarsdale Road
Tuckahoe New York(US)

(72) Inventor: Musser, John H.
1009 Millstream Road
Malvern Pennsylvania(US)

(72) Inventor: Sutherland, Charles A.
222 Elwood Avenue
Hawthorne New York(US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

(54) Benzoheterocyclic carbamates.

(57) Compounds of the structure;

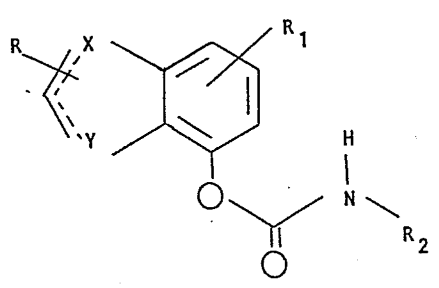

and pharmaceutically acceptable salts thereof, wherein:

R and $R_1$ are each H, alkyl, alkoxy, carboxy, alkylcarboxy, trifluoromethyl, halogen, nitro or hydroxy;

X is O, N, S or C;

Y is O, N or S; and

$R_2$ is H, alkyl, cycloalkyl, aryl or heteroaryl useful in the treatment of ischemic heart disease and hypertriglyceridemia.

EP 0 116 938 A1

PATENTANWÄLTE
GR...ECKER · DR. KINKELDEY · DR. STOCKMAIR
DR. SCHUMANN · JAKOB · DR. BEZOLD · MEISTER
HILGERS · ...

0116938

# BENZOHETEROCYCLIC CARBAMATES

We have found that benzoheterocyclic carbamates are active antilipolytic agents as evidenced by the myocardial lipase and the rat adipocyte assays.

Lipolysis is associated with ischemic heart disease: free fatty acid has a detrimental effect on the ischemic heart by disrupting electrical conduction, decreasing myocardial efficiency and preventing the transfer of adenosine diphosphate and adenosine triphosphate, in and out, respectively, of the mitochondria. Interventions which depress myocardial oxygen consumption in animals and man provide a protective effect against ischemic injury.

This invention provides compounds capable of inhibiting lipolysis associated with ischemic heart disease.

It has been found that benzoheterocyclic carbamates are active antilipolytic agents, while the structurally related compounds, 8-quinoline carbanilates, are known in the prior art as fungicides, pesticides, amebicides and bactericides.

Compounds of the Structure

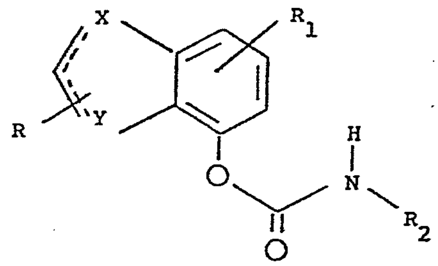

and pharmaceutically acceptable salts thereof, wherein:

R and $R_1$ are each H, alkyl, alkoxy, carboxyl, carbalkoxy, trifluoromethyl, halogen, nitro or hydroxy;

X is O, N, S or C;

Y is O, N or S; and

$R_2$ is H, alkyl, cycloalkyl, aryl, or heteroaryl,

are useful in the treatment of ischemic heart disease and hypertriglyceridemia.

The alkyl group and the alkyl moieties in alkoxy and carbalkoxy contain from 1 to 10 carbon atoms and may be a straight or a branched chain. Such groups include for example methyl, ethyl, propyl, isopropyl, butyl, isobutyl, amyl, and isoamyl.

The halogen is F, Cl, Br or I.

The aryl group may be unsubstituted or substituted with up to 3 of the following substituents: alkyl, alkoxy, carboxyl, alkylcarboxy, trihalomethyl, halogen, nitro or hydroxy.

The heteroaryl is selected from the group consisting of tetrazolyl, pyridyl, furanyl, thienyl or imidazolyl.

The compounds of this invention are prepared by condensing a hydroxy compound of formula II

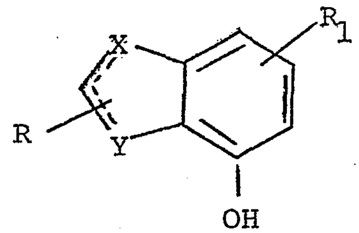

(II)

with an isocyanate $R_2$ NCO in the classical manner to form the corresponding carbamate compound of formula I herein. The substituents R, $R_1$, X, Y and $R_2$ are as previously defined except that R, $R_1$ and $R_2$ should not include groups which are reactive with the aforesaid isocyanate under the reaction conditions employed. Such reactive groups should be blocked, preferably with groups that are readily removable after carbamate formation if the reactive groups are desired on the final product. Blocking groups include, for example, acyl groups, e.g. acetyl, or ether groups such as methyl or benzyl which can be readily removed after carbamate product formation as is well-known in the art.

The reaction is accomplished by merely contacting the reactants preferably in an organic solvent and in the presence of a base such as an organic tertiary amine e.g. pyridine, triethylamine, and similar amines, conveniently at room temperature, although temperatures from 0°C to the reflux temperature of the reaction mixture can be used. The use of high temperatures will materially shorten the reation time but may lead to secondary reactions and is therefore not

preferred. The reaction can be followed by known methods using aliquots of the reaction mixture to determine the amounts of isocyanate still present. Usually, the reaction is allowed to proceed for from about one hour to several hours to assure completeness of reaction.

The product is isolated from the reaction mixture using standard methods, e.g. evaporation of the solvent, and purification by chromatographic techniques.

Schematic procedures for the preparation of compounds of the present invention follow:

1.

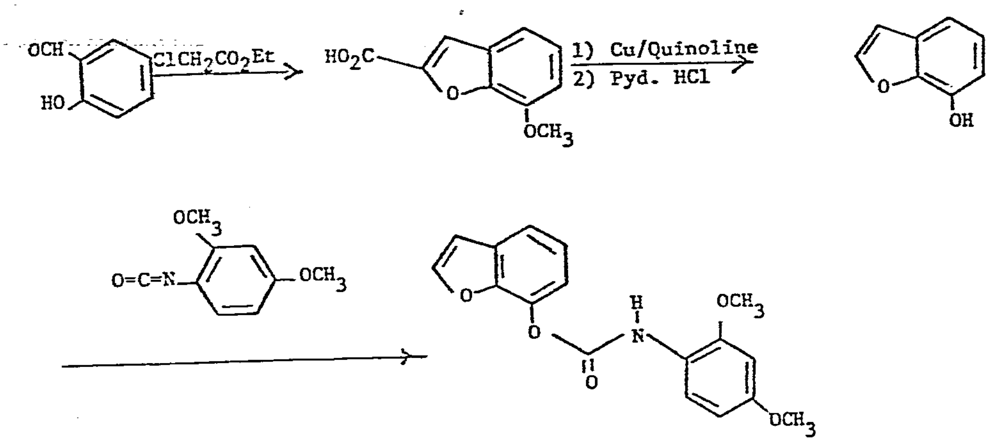


2.

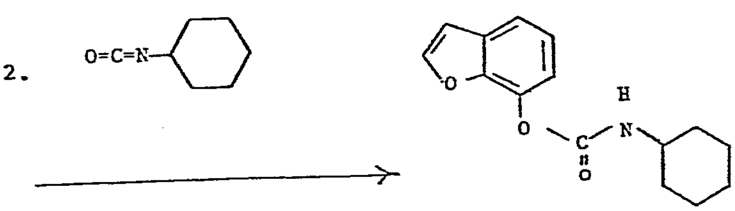

3.

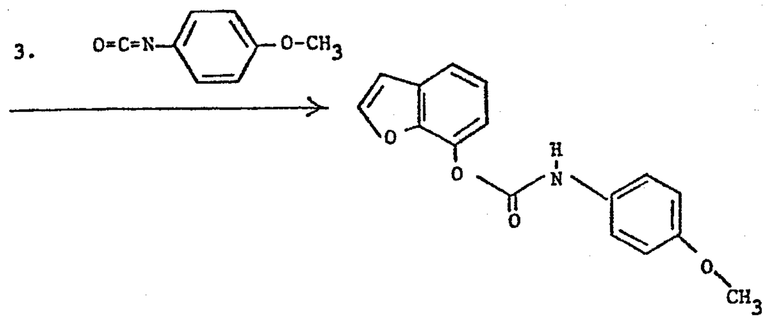

4.

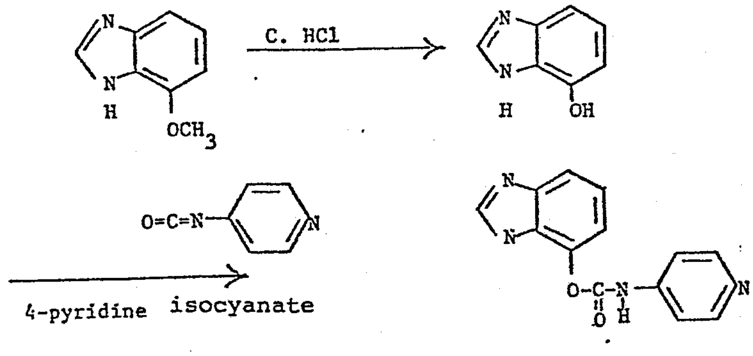
C. HCl
4-pyridine isocyanate

5.

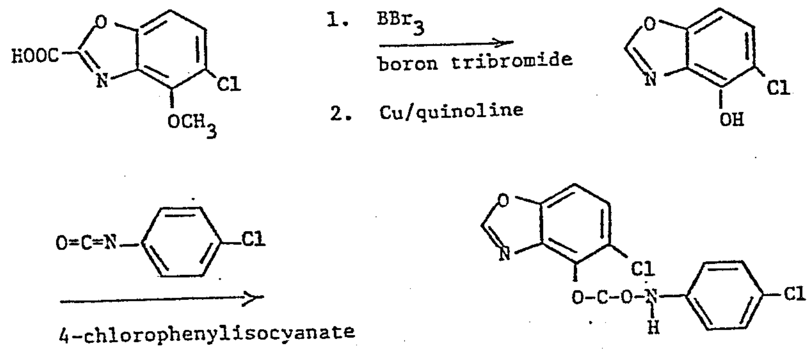
1. $BBr_3$
boron tribromide
2. Cu/quinoline
4-chlorophenylisocyanate

6.

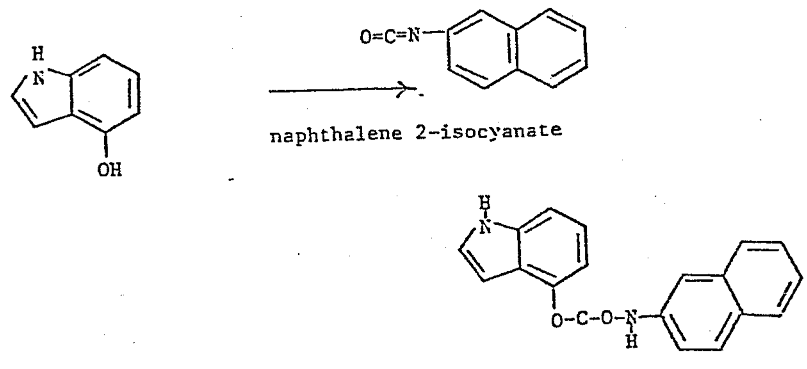
naphthalene 2-isocyanate

7. 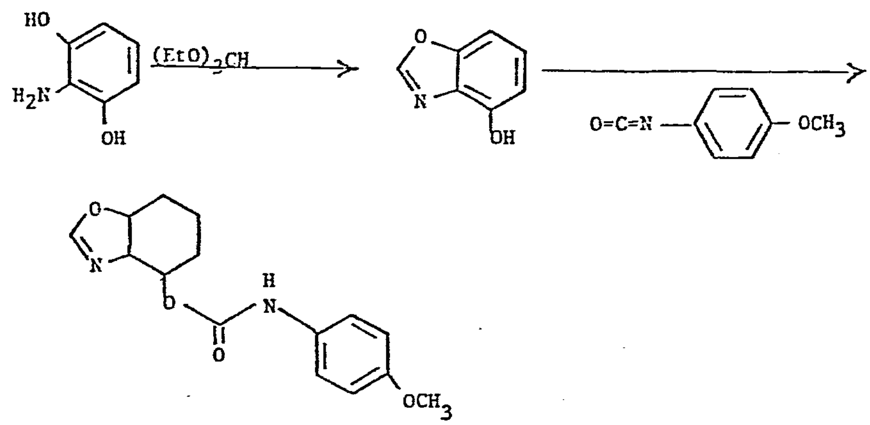


The invention will be more fully illustrated in the examples that follow. These examples are given by way of illustration and are not to be considered as limiting.

<u>Example 1A</u>

<u>7-Methoxybenzofuranyl-2-carboxylic acid</u>

A mixture of 42 g. 2-vanillin (Aldrich 12080-4), 45 g. -chloroethylacetate (Pfaltz & Bauer C12130), 75 g. potassium carbonate and 500 ml DMF was heated at 80°C for 4 hours. The reaction was poured into ice and extracted with methylene chloride. The aqueous layer was acidified with hydrochloric acid and a precipitate formed. The precipitate was filtered and dried giving 27 g. (46% yield) of solid, m.p. 200°C.

0116938

Example 1B

7-Methoxybenzofuran

To a solution of 7-methoxybenzofuranyl-2-carboxylic acid (2.6 g.) in quinoline (10 ml) was added finely ground copper metal. The reaction was slowly heated until the evolution of gas was noted. After termination of gas evolution, the mixture was cooled and diluted with methylene chloride. The solution was washed with 5% aqueous hydrochloric acid (six times) ; dried ($MgSO_4$) and concentrated to an oil, 1.8 g., (90% yield).

Example 1C

7-Hydroxybenzofuran

A mixture of 7-methoxybenzofuran (1.8 g.) and pyridine hydrochloride (4.6 g.) was heated at 220° for 2-1/2 hours. The reaction was cooled and diluted with methylene chloride. The solution was washed with 5% aqueous hydrochloric acid (six times) ; dried ($MgSO_4$) and concentrated to an oil, 0.8 g. (50% yield).

Example 1D

4-Hydroxybenzoxazole

A mixture of 2.1 g. 2-aminoresorcinol (Pfaltz & Bauer, A28620) , 3.7 g. triethylformate and 0.1 g. sulfuric acid was heated at 130°C for 30 minutes. After cooling, the remaining solid was recrystallized from ethyl acetate, 1.4 g. (62% yield).

Example 1E

7-Benzofuranyl-4-methoxy carbanilate

To a solution of 7-hydroxybenzofuran (0.7 g.) in ethyl ether (25 ml) and triethylamine (1 ml) was added 0.8 g. 4-methoxyphenyl isocyanate (Aldrich 23860-0). The reaction was stirred at room temperature for 3 days. The precipitate which forms was filtered and dried, 0.8 g. (54% yield) ; m.p. 157-159°C.

Examples 2-4

In like manner as above, using appropriate starting materials and reagents, the following compounds were prepared:

7-Benzofuranyl-2, 4-dimethoxy carbanilate; m.p. 102-103°C.

7-Benzofuranyl-cyclohexyl carbanilate; m.p. 149-151°C.

4-Benzoxazoyl-4-methoxy carbanilate; m.p. 161-162°C.

Other benzoheterocyclic carbanilates can be made as illustrated by the following examples:

Example 5A

2-Nitro-3-benzyloxyphenylypyruvic acid

A solution of 50 g. 3-hydroxy-2-nitrotoluene (Pfaltz & Bauer, NO9200) , and 30 ml benzyl bromide in acetone is treated with excess potassium carbonate and refluxed for 2-1/2 hours. The reaction is filtered and the solvent is removed in vacuo. The resulting ether is distilled. This compound (24 g.) is added to a solution of potassium ethoxide (4 g. potassium in ethanol) in ethyl ether (350 ml) and refluxed for 18 hours. The solvent is removed in vacuo and the remaining oil is treated with 1 N NaOH at room temperature for 1 hour. The mixture is washed with ether (twice), acidified with hydrochloric acid yielding the desired product.

Example 5B

7-Hydroxyindole

Reaction of 2-nitro-3-benzyloxyphenylpyruvic acid (2 g.) with 10% palladium on carbon (2 g.) in acetic acid under hydrogen atmosphere is complete in 30 minutes. The mixture is filtered and the solvent is removed in vacuo. The remaining material is dissolved in glycerol and heated at 220°C for 20 minutes. The desired product is obtained by sublimation under high vacuum.

Example 5C

7-Chloro-3-methylbenzo [b] thiophene

To a solution of 11.8 g. 2-chlorothiophenol (Aldrich, 155667) dissolved in aqueous sodium hydroxide (4.0 g./50 ml water) is added chloracetone (9.3 g.). After heating for 1 hour the reaction is cooled and extracted with methylene chloride. The extract is dried ($MgSO_4$) and concentrated to an oil. The oil is added to polyphosphoric acid (100 g.) and slowly heated to 120°C. The mixture is added to ice and extracted with ethyl ether. The ether extract is washed with water (twice) ; dried ($MgSO_4$) and concentrated to an oil. The oil is purified by distillation.

Example 5D

7-Hydroxy-3-methylbenzo [b] thiophene

Cyclohexyl bromide (32.6 g.) and 7-chloro-3-methylbenzo [b] thiophene (18.3 g.) in THF (400 ml) is added to a suspension of magnesium turnings (7.3 g.) in THF. The mixture is stirred for two hours and heated on a steam bath for two hours. The reaction is cooled and oxygen is bubbled in slowly for two hours. The mixture is stirred for 18 hours at room temperature and extracted with chloroform. The organic extract is extracted with 5% sodium hydroxide. The aqueous extract is acidified with concentrated hydrochloric acid and extracted with ethyl ether. The ether extract is dried ($MgSO_4$) and concentrated to a solid.

Example 5E

7-Hydroxybenzoxazole

A mixture of 2,3-dihydroxyaniline (2.5 g.) , triethylorthoformate (4.5 g.) and 0.1 g. sulfuric acid is heated at 130°C for one hour. After cooling, the remaining material is purified by HPLC on silica gel.

Examples 5F-5H

In like manner as above, using the appropriate starting materials and reagents, the following compounds can be prepared:

4-hydroxybenzthiazole
7-Hydroxybenzthiazole
1-methyl-4-hydroxybenzimidazole

Example 5I

7-Indolyl-4-methoxy carbanilate

To a solution of 7-hydroxyindole (0.7 g.) in ethyl ether (25 ml) and triethylamine (1 ml) is added 0.8 g. 4-methoxyphenyl isocyanate (Aldrich 23860-0). The reaction is stirred for 3 days at room temperature. The solvent is removed *in vacuo* and the remaining oil is purified by HPLC on silica gel.

0116938

Examples 6-22

In like manner as above, using appropriate starting materials and reagents, the following compounds are prepared:

7-indolyl-4-methyl carbanilate
7-indolyl-3-trifluoromethyl carbanilate
7-indolyl-3-nitro carbanilate
7-indolyl-2-chloro carbanilate
7-indolyl-2-bromo carbanilate
3-methyl-7-benzo [b] thiophenolyl-4-methoxy carbanilate
3-methyl-7-benzo [b] thiophenolyl carbanilate
3-methyl-7-benzo [b] thiophenolyl-4-dimethylamino carbanilate
3-methyl-7-benzo [b] thiophenolyl-3-acetyl carbanilate
7-benzoxazolyl-4-methoxy carbanilate
7-benzoxazolyl-cyclopentyl carbanilate
4-benzothiazolyl-4-methoxy carbanilate
4-benzothiazolyl-2-cyano carbanilate
7-benzothiazolyl-4-methoxy carbanilate
7-benzothiazolyl-4-acetamido carbanilate
1-methyl-7-benzimidazolyl-4-methoxy carbanilate
1-methyl-7-benzimidazolyl-3-carbomethoxy carbanilate

The compounds of the present invention exhibit activity in the myocardial lipase assay, the rat adipocyte assay and the *in vivo* lipolysis assay.

Myocardial Lipase Assay

All compounds were dissolved in DMSO (final concentration 3.0%) and tested in duplicate at a concentration of 100 mM. Canine cardiac lipases were obtained by extracting washed heart membranes with buffer plus heparin and a small amount of Triton X-100 detergent. Because these enzymes are only active at an oil-water interface, the enzyme reaction is run in an oil-water emulsion that contains triolein substrate, Tris buffer (50mM, pH 6.8) and a small amount of bovine serum albumin (0.5%) added to stabilize the emulsion. A small amount of tritiated triolein was added to the unlabelled triolein substrate. Tritium-labelled oleic acid released by the lipases was extracted into hexane, separated from unreacted triolein and counted in scintillation counter. Inhibitory agents reduce the amount of radioactivity appearing in the free fatty acid fraction isolated in the extraction procedure.

## Rat Adipocyte Assay

Abdominal fat pads were removed from male rats weighing 200-250 grams and placed in Krebs-bicarbonate buffer gassed with 95% $O_2$/5% $CO_2$. The fat pads were digested with collagenase for 1 hour at 37°C, washed twice with Krebs-bicarbonate buffer and distributed among a set of 20 ml plastic counting vials. Two such vials received only buffer and cells (4ml) but no agonists or antagonists. The remaining vials received epinephrine (3μM) plus the phosphodiesterase inhibitor methylisobutylxanthine (10 μM). Test compounds were dissolved in DMSO or water to a concentration of 20 μM and 40 μl was added to the buffer plus cells in the counting vials. The final compound concentration for routine screening was 200 μM (final concentration of DMSO = 1%).

The cells were incubated for 1 hour at 37°C under a 95% $O_2$/5% $CO_2$ atmosphere. The incubation was stopped by placing the vials in crushed ice. The cells and medium were transferred to test tubes, centrifuged and the cell layer removed by aspiration. The aqueous phase was assayed for glycerol using the enzyme glycerol dehydrogenase.

The glycerol dehydrogenase assay for glycerol depends on the enzyme catalyzed conversion of glycerol to glyceraldehyde and NAD to NADH. The assay can detect as little as 5-10 nanomoles of glycerol. The aqueous phase, following removal of cells, usually contained about 50 to 80 nanomoles of glycerol per 300 μl of assayed sample if no inhibitory activity was present. Samples from the control tubes (no agonist) usually contained 0-5 nanomoles of glycerol per 300 μl of sample.

In Vivo Lipolysis Assay

This assay is based on the general concepts described by Lovisolo et al. Lovisolo, P. P., Briatico-Vangosa, G., Orsini, G., Ronchi, R. and Angelucci, R. Pharmacological profile of a new antilipolytic agent: 5-methylpyrazine-2-carboxylic acid-4-oxide (Acipimox) II - antilipolytic and blood lipid lowering activity. Pharmacological Research Communications 13;163-174(1981).

Sixteen male rats (200-300 grams) are marked with a color code to facilitate individual identification during the course of the experiment. Each assay is to include 4 nonfasted control animals.

The remaining animals are fasted for 22 hours (water *ad libitum*). Test compounds are prepared either in methocel (S-2) or methocel plus DMSO (S-3). After 22 hours of fasting the animals are injected i.p. at five minute intervals with either 2 ml of carrier solution (S-2 or 3) or 2 ml of carrier solution plus test compound. This procedure is carried out on animals lightly anesthetized with ether.

Two hours after drug injection, at 5 min intervals, the animals are again lightly anesthetized and the abdominal cavity is opened. Blood is withdrawn from the inferior vena cava through a 16 gauge needle into a 20 ml syringe. No anticoagulant is used. The blood is transferred immediately to 12 ml plastic centrifuge tubes. The blood is allowed to coagulate at room temperature for 1 hour. The tubes are then centrifuged at 4000 x g (3000 rpm) for 10 min in an RC-3 Sorval refrigerated centrifuge. The clear serum (3-4 ml/sample) is transferred to the siliconized vacutainer tubes, labeled and stored at -70°C. The serum samples are analyzed for determination of unesterified fatty acids.

The results obtained on representative compounds of the present invention are shown in Table I.

Table I: INHIBITION OF MYOCARDIAL LIPASE, RAT ADIPOCYTE LIPOLYSIS AND IN VIVO LIPOLYSIS BY BENZOHETERO-CYCLIC CARBANILATES

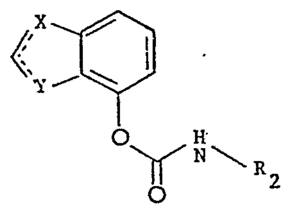

| # | X | Y | $R_2$ | Lipase $I_{50}$ µM | Adipocyte $I_{50}$µM | In Vivo Lipolysis % Inhib. at mg/Kg |
|---|---|---|---|---|---|---|
| 1 | CH | O | 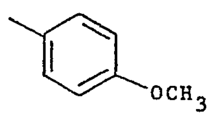 | 16 | 0.3 | 97 @ 25 p.o. |
| 2 | CH | O | 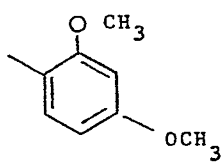 | 40 | 0.3 | 71@ 100 p.o. |
| 3 | CH | O | 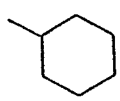 | 10 | 12.0 | 26@100 p.o. |
| 4 | O | N | 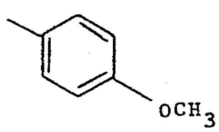 | 79 | 2.4 | 40@100 i.p. |

The therapeutic compounds of this invention may be administered to a mammal alone or in combination with pharmaceutically acceptable carriers, the proportion of which is determined by the solubility and chemical nature of the compound, chosen route of administration and standard pharmaceutical practice.

The physician will determine the dosage of the present therapeutic agents which will be most suitable and it will vary with the form of administration and the particular compound chosen, and also, it will vary with the particular patient under treatment. He will generally wish to initiate treatment with small dosages substantially less than the optimum dose of the compound and increase the dosage by small increments until the optimum effect under the circumstances is reached. The therapeutic dosage will generally be from several mg to about 30 mg/kg of body weight or higher.

0116938

What is claimed is:

1. A compound of the formula

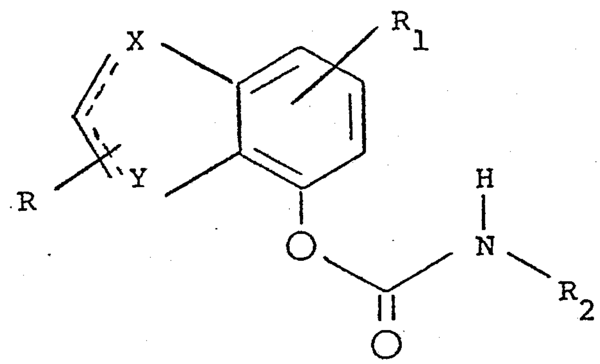

and salts thereof, especially pharmaceutically acceptable salts thereof, wherein:

R and $R_1$ are each H, alkyl, alkoxy, carboxy, carbalkoxy, trifluoromethyl, halogen, nitro or hydroxy;

X is O, N, S, or C;

Y is O, N or S; and

$R_2$ is H, alkyl, cycloalkyl, aryl, or heteroaryl.

2. The compound of claim 1 wherein the alkyl group and the alkyl moieties in alkoxy and carbalkoxy contain from 1 to 10 carbon atoms.

3. The compound of claim 1 wherein said alkyl group and alkyl moieties in alkoxy and carbalkoxy is methyl, ethyl, propyl, isopropyl, butyl, isobutyl, amyl or isoamyl.

4. The compound as in any of claims 1-3 wherein said aryl group is substituted with up to three members selected from the group consisting of H, alkyl, alkoxy, carboxyl, alkylcarboxy, trifluoromethyl, halogen, nitro or hydroxy.

5. The compound as in any of claims 1-3 wherein said heteroaryl is selected from the group consisting of tetrazolyl, pyridyl, furanyl, thienyl or imidazolyl.

6. The compound as in claim 1 which is 7-Benzofuranyl-4-methoxy carbanilate.

7. The compound as in claim 1 which is 7-Benzofuranyl-2, 4-dimethoxy carbanilate.

8. The compound as in claim 1 which is Benzofuranyl-cyclohexyl carbanilate.

9. The compound as in claim 1 which is 4-Benzoxazoyl-4-methoxy carbanilate.

10. A therapeutic composition comprising an effective amount of a compound as in any of claims 1-9 and a pharmaceutically acceptable carrier therefor.

European Patent Office

**EUROPEAN SEARCH REPORT**

0116938
Application number

EP 84 10 1493

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| X | US-A-3 564 605 (FMC) * Claim 2; example 1 * --- | 1 | C 07 D 307/86<br>C 07 D 263/56<br>C 07 D 209/08<br>C 07 D 333/54<br>A 61 K 31/325 |
| X | GB-A-1 101 415 (FISONS PEST CONTROL) * Page 2, line 25; example 4; example 7; claims 1,13 * --- | 1-3 | |
| X | FR-A-2 107 714 (BAYER) * Formula IV; page 2 * --- | 1-3 | |
| X | FR-A-1 481 940 (MOBIL OIL) * Page 4, abstract, part A; example 1 * --- | 1-3 | TECHNICAL FIELDS SEARCHED (Int. Cl. 3) |
| X | US-A-3 288 673 (MOBIL OIL) * Column 1, lines 32-55; example 13; claim 4 * --- | 1-3 | C 07 D 307/00<br>C 07 D 263/00<br>C 07 D 209/00<br>C 07 D 333/00<br>A 61 K 31/00 |
| X | GB-A-1 220 056 (FISONS PEST CONTROL) * Page 1, lines 8-19; example 8; claim 1 * ----- | 1-3 | |

The present search report has been drawn up for all claims

| Place of search | Date of completion of the search | Examiner |
|---|---|---|
| THE HAGUE | 17-05-1984 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82